# EUROPEAN PATENT SPECIFICATION

(11) **EP 2 217 246 B1**
(45) Date of publication and mention of the grant of the patent: **17.12.2014**
(21) Application number: 08848466.2
(22) Date of filing: 07.11.2008
(51) Int. Cl.: A61K 31/7028, A23L 1/29, A23L 1/30, A23L 1/308, A61P 37/04, A61P 37/08, A61K 45/06, A23L 1/09, A61K 35/74

(54) **USE OF OLIGOSACCHARIDES CONTAINING N-ACETYLLACTOSAMINE FOR MATURATION OF IMMUNE RESPONSES IN NEONATES**
VERWENDUNG VON OLIGOSACCHARIDEN ENTHALTEND N-ACETYL-LACTOSAMIN ZUR MODULATION DER IMMUNREAKTION VON NEUGEBORENEN
UTILISATION DU N-ACÉTYL-LACTOSAMINE OU D'OLIGOSACCHARIDES CONTENANT DU N-ACÉTYL-LACTOSAMINE POUR LA MODULATION DE RÉPONSES IMMUNES CHEZ LES NOUVEAUX-NÉS

(30) Priority: 08.11.2007 EP 07120265
(43) Date of publication of application: 18.08.2010
(73) Proprietor: Nestec S.A., 1800 Vevey (CH)
(72) Inventor: FICHOT, Marie-Claire, CH-1807 Blonay (CH); SPRENGER, Norbert, CH-1073 Savigny (CH)
(74) Representative: Fraisse, Sandrine
(86) International application number: PCT/EP2008/065143
(87) International publication number: WO 2009/060073

(56) References cited:
- EP-A- 1 062 873
- WO-A-98/43494
- WO-A-98/43495
- WO-A-02/056893
- WO-A-2004/002495
- WO-A-2004/112509
- WO-A-2005/067962
- WO-A-2006/113035
- WO-A-2007/104311
- WO-A-2007/105945
- US-A- 4 919 961
- US-A1- 2003 129 278
- US-A1- 2005 004 070
- US-B1- 6 204 431
- ANONYMOUS: "Aptamil" INTERNET CITATION, [Online] 16 January 2004 (2004-01-16), XP002412131 Retrieved from the Internet: URL:http://www.milupa.ch/images/prebiotica _pressemitteilung.pdf> [retrieved on 2006-12-19]
- KUNZ C: "Complex oligosaccharides in infant nutrition" MONATSSCHRIFT FUR KINDERHEILKUNDE 1998 DE, vol. 146, no. 8 SUPPL. 1, 1998, pages S49-S56, XP002473723 ISSN: 0026-9298
- RINNE ET AL: "Effect of Probiotics and Breastfeeding on the Bifidobacterium and Lactobacillus/Enterococcus Microbiota and Humoral Immune Responses", JOURNAL OF PEDIATRICS, MOSBY-YEAR BOOK, ST. LOUIS, MO, US, vol. 147, no. 2, 1 August 2005 (2005-08-01), pages 186-191, XP005692247, ISSN: 0022-3476, DOI: 10.1016/J.JPEDS.2005.03.053

## Description

### Field of the Invention

This invention relates to the maturation of immune responses of neonatal infants by education of the immune system to avoid inappropriate immune responses. The invention is defined by the claims.

### Background to the Invention

Immediately before birth, the gastro-intestinal tract of a baby is thought to be sterile. During the normal process of birth, it encounters bacteria from the digestive tract, skin and environment of the mother and starts to become colonised. The faecal microbiota of a healthy breast-fed infant of age 2 to 4 weeks which may be taken as the optimum microbiota for this age group is dominated by Bifidobacteria species with some Lactobacillus species and lesser amounts of Bacteroides such as *Bacteroides fragilis* species, to the exclusion of potential pathogens such as Clostridia. After the completion of weaning at about 2 years of age, a pattern of gut microbiota that resembles the adult pattern becomes established.

The early stages of colonisation is a critical phase of adaptation to the gut's new environment, which provides most of the identified innate immune receptor ligands, such as lipopolysaccharides, at high concentrations. Gut innate immune recognition must therefore be closely regulated and educated during this period in order to avoid inappropriate stimulation.

In mice, intestinal epithelial cells respond within several hours of birth to exogenous bacterial compounds, such as endotoxins, to acquire toll like receptor tolerance to facilitate subsequent microbial colonization and the development of a stable intestinal host-microbe homeostasis (Lotz et al., 2006). It is hypothesised that similar postnatal processes also occur in human neonates. Dysfunction of the down-regulation of proinflammatory reactions which is necessary to allow colonisation to take place may lead to the development of enteric inflammation and disturbances in the colonisation process. This in turn could lead to a long-lasting battle between host and microbes, with negative consequences for health in the short and likely also at the long term both locally in the gut and even systemically.

It has already been suggested that this delayed or inappropriate colonisation may have specific consequences in terms of the subsequent development of the infant. For example, Fantuzzi et al have proposed that systemic low-grade inflammation and a sub-optimal gut microbiota may be implicated in the later development of obesity (Fantuzzi G. "Adipose tissue, adipokines, and inflammation" J Allergy Clin Immunol. 2005; 115:911-919).

In short, more and more evidence is emerging which suggests that the establishment of an appropriate intestinal microbiota early in life may be a significant factor in subsequent healthy development. Moreover, an inappropriate intestinal microbiota is likely to be accompanied by local inflammation in the gut and low-level systemic inflammation which has its own adverse consequences on general health.

Mother's milk is recommended for all infants. In addition to macro-nutrients, human milk is known to contain many bioactive nutrients, that is, compounds which may be categorised in broad terms as proteins, carbohydrate or fats but which have a specific function over and above their calorific value. One large class of such bioactive nutrients is human milk oligosaccharides, a group of over 100 oligosaccharides which are found in varying quantities in human milk but not in the same quantity or variety in bovine milk for example. The number and function of these various oligosaccharides are still being elucidated although certain of them have already been associated with, for example, reducing the ability of pathogens to adhere to host epithelial cells.

Therefore, supplementation with oligosaccharides was suggested. A galactose-containing saccharide, i.e. lacto-N-neotetraose, is shown in WO 98/43495 to stimulate the metabolic activity of *Bifidobacterium lactis.* Enteral nutritional compositions containing lacto-N-neotetraose are thus suggested for feeding infants in order to inhibit infections with *Bacteroides, Clostridium* and *E. coli.* By inhibiting the growth of these bacteria, infants are supposed to be provided with resistance against gastroenteritis.

In order to improve the immune system of infants, it is suggested in WO 2007/105945 administering water-soluble, non-digestible galactose containing saccharides to their mothers during pregnancy. Supplementation with such saccharides is reported to increase the percentage of bifidobacteria and lactobacilli in the gut microflora of the pregnant woman. As a result, inoculation of the intestine of the infant during birth and colonization after birth with lactobacilli and bifidobateria is improved.

### Summary of the Invention

The invention is defined by the claims.

The present inventors have surprisingly found that administration of oligosaccharides with lactose bound mono-, di- and/or poly-valent N-acetyl-lactosamine structures to the neonatal infant is particularly effective in the education of the immune system, promoting down-regulation of inappropriate pro-inflammatory responses during the establishment of the intestinal microbiota in the neonatal period with the result that inappropriate immune responses can likewise be down-regulated.

Accordingly, the use of an oligosaccharide selected from the group consisting of lacto-N-tetraose, lacto-N-neotetraose, lacto-N-hexaose, lacto-N-neohexaose, para-lacto-N-hexaose, para-lacto-N-neohexaose, lacto-N-octaose, lacto-N-neooctaose, iso-lacto-N-octaose, para-lacto-N-octaose and lacto-N-decaose in the manufacture of a medicament for infants or a therapeutic nutritional composition for infants for maturation of immune responses in a neonatal infant is disclosed.

In one aspect, the invention provides the use of an oligosaccharide selected from the group consisting of lacto-N-tetraose, lacto-N-neotetraose, lacto-N-hexaose, lacto-N-neohexaose, para-lacto-N-hexaose, para-lacto-N-neohexaose, lacto-N-octaose, lacto-N-neooctaose, iso-lacto-N-octaose, para-lacto-N-octaose and lacto-N-decaose in the manufacture of a medicament for infants or a therapeutic nutritional composition for infants for modulating the immune system of a neonatal infant to promote the development in the first few weeks of the life of the infant of a beneficial intestinal microbiota comparable with that found in breast fed infants, wherein the medicament or therapeutic nutritional composition is administered to the infant immediately after delivery and thereafter for at least 2 months.

In a further aspect, the invention provides the use of an oligosaccharide selected from the group consisting of lacto-N-tetraose, lacto-N-neotetraose, lacto-N-hexaose, lacto-N-neohexaose, para-lacto-N-hexaose, para-lacto-N-neohexaose, lacto-N-octaose, lacto-N-neooctaose, iso-lacto-N-octaose, para-lacto-N-octaose and lacto-N-decaose in the manufacture of a medicament or therapeutic nutritional composition for administration to a neonatal infant for reducing the risk of subsequent development of allergy in enfant, wherein the medicament or therapeutic nutritional composition is administered to the infant immediately after delivery and thereafter for at least 2 months.

Also disclosed is a method of promoting the maturation of immune responses in a neonatal infant by administering to a neonatal infant in need thereof a therapeutic amount of an oligosaccharide selected from the group consisting of lacto-N-tetraose, lacto-N-neotetraose, lacto-N-hexaose, lacto-N-neohexaose, para-lacto-N-hexaose, para-lacto-N-neohexaose, lacto-N-octaose, lacto-N-neooctaose, iso-lacto-N-octaose, para-lacto-N-octaose and lacto-N-decaose.

Further disclosed is a method of promoting the development in the first few weeks of the life of an infant of a beneficial intestinal microbiota comparable with that found in breast fed infants by administering to a neonatal infant in need thereof a therapeutic amount of an oligosaccharide selected from the group consisting of lacto-N-tetraose, lacto-N-neotetraose, lacto-N-hexaose, lacto-N-neohexaose, para-lacto-N-hexaose, para-lacto-N-neohexaose, lacto-N-octaose, lacto-N-neooctaose, iso-lacto-N-octaose, para-lacto-N-octaose and lacto-N-decaose.

Finally disclosed is a method of reducing the risk of subsequent development of allergy in an infant by administering to a neonatal infant in need thereof a therapeutic amount of oligosaccharide selected from the group consisting of lacto-N-tetraose, lacto-N-neotetraose, lacto-N-hexaose, lacto-N-neohexaose, para-lacto-N-hexaose, para-lacto-N-neohexaose, lacto-N-octaose, lacto-N-neooctaose, iso-lacto-N-octaose, para-lacto-N-octaose and lacto-N-decaose.

Without wishing to be bound by theory, the present inventors believe that oligosaccharides with lactose bound mono-, di- and/or poly-valent N-acetyl-lactosamine structures in the intestinal lumen may act as a "decoy" for pro-inflammatory factors preventing them from binding to their natural ligands and thereby alleviating pro-inflammatory responses. Specifically, galectin-3 is a soluble ß-galactoside-binding protein involved in various pro-inflammatory reactions, such as activation of neutrophils (e.g. IL8). Neutrophils participate in the innate immune response as a major phagocytic leukocyte recruited to infection sites. Galectin-3 can be released from gut epithelia to activate the immune system.

Studies with galectin-3 null mice (gal-/-) showed the regulatory role of galectin-3 to tune both innate and adaptive immune responses to pathogens (Bernardes et al., 2006). With a similar pathogen burden, gal-/- mice showed much lower inflammatory response, lower leukocyte infiltration into gut lamina propria and no gut necrosis as compared to the wildtype controls. Thus it can be concluded that absence of galectin-3 leads to milder inflammatory response.

Glycans terminating with galactose ß-1,4 N-acetylglucosamine such as lacto-N-neotetraose, bind galectin-3. As previously shown for galectin-9, free galectin-binding glycans can interfere with galectin binding to target ligands thereby altering target ligand behaviour (Ohtsubo et al., 2005). The more N-acetyllactosamine units are polymerized the higher the affinity for galectin-3 and the stronger the decoy function

Further, gal3-/- mice showed a higher Th1 response manifested by higher IL12 and IFN-γ production by dendritic cells (Bernardes et al., 2006). T helper cells play a central role in adaptive immunity. Th1 cells are vital for cell-mediated immune responses, and Th2 cells promote humoral immunity. Th1 and Th2 responses are counter-regulative, that is, cytokines produced by Th1 cells inhibit Th2 function and vice versa. Th2-skewed immune response has been shown to be crucial for the maintenance of successful pregnancy and it also prevails at birth and during the first months of life. Postnatal exposure to microbial antigens elicits preferentially Th1 responses, which have been suggested to counterbalance Th2-polarized cytokine production in neonates. In the case of insufficient early Th1 responses, the production of Th2-type cytokines (IL-4, IL-5 and IL-13) is further propagated leading to IgE production and consequently to allergic disease. It follows that the decoy activity described above also helps to bias the immune system towards a Th1 response thus educating the immune system and reducing the risk of development of allergy early in life.

### Detailed Description of the Invention

In this specification, the following terms have the following meanings:-
"beneficial intestinal microbiota comparable with that found in breast fed babies" means an intestinal microbiota dominated by appreciable populations of Bifidobacterium and Lactobacillus species to the exclusion of appreciable populations of such species as Bacteroides, Clostridia and Streptococci;
"neonatal infant" means an infant in the first two months of life.

All references to percentages are percentages by weight unless otherwise stated.

The invention relates to the use of an oligosaccharide selected from the group consisting of lacto-N-tetraose, lacto-N-neotetraose, lacto-N-hexaose, lacto-N-neohexaose, para-lacto-N-hexaose, para-lacto-N-neohexaose, lacto-N-octaose, lacto-N-neooctaose, iso- lacto-N-octaose, para- lacto-N-octaose and lacto-N-decaose. Preferred oligosaccharides are lacto-N-tetraose (LNT) and lacto-N-neotetraose (LNnT). LNT and LNnT may be synthesised chemically by enzymatic transfer of saccharide units from donor moieties to acceptor moieties using glycosyltransferases as described for example in US Patent No. 5,288,637. Alternatively, LNT and LNnT may be prepared by chemical conversion of ketohexoses (e.g. fructose) either free or bound to an oligosaccharide (e.g. lactulose) into N-acetylhexosamine or an N-acetylhexosamine containing oligosaccharide as described in Wrodnigg, T.M.; Stutz, A.E. (1999) Angew. Chem. Int. Ed. 38:827-828. N-acetyllactosamine produced in this way may then be transferred to lactose as acceptor moiety.

The therapeutic nutritional composition is preferably an infant formula which contains an oligosaccharide selected from the group consisting of lacto-N-tetraose, lacto-N-neotetraose, lacto-N-hexaose, lacto-N-neohexaose, para-lacto-N-hexaose, para-lacto-N-neohexaose, lacto-N-octaose, lacto-N-neooctaose, iso- lacto-N-octaose, para- lacto-N-octaose and lacto-N-decaose in an amount between 0.1 and 3g/100g composition on a dry weight basis.

An infant formula for use according to the present invention may contain a protein source in an amount of not more than 2.0 g/100kcal, preferably 1.8 to 2.0 g/100kcal. The type of protein is not believed to be critical to the present invention provided that the minimum requirements for essential amino acid content are met and satisfactory growth is ensured although it is preferred that over 50% by weight of the protein source is whey. Thus, protein sources based on whey, casein and mixtures thereof may be used as well as protein sources based on soy. As far as whey proteins are concerned, the protein source may be based on acid whey or sweet whey or mixtures thereof and may include alpha-lactalbumin and beta-lactoglobulin in whatever proportions are desired.

The proteins may be intact or hydrolysed or a mixture of intact and hydrolysed proteins. It may be desirable to supply partially hydrolysed proteins (degree of hydrolysis between 2 and 20%), for example for infants believed to be at risk of developing cows' milk allergy. If hydrolysed proteins are required, the hydrolysis process may be carried out as desired and as is known in the art. For example, a whey protein hydrolysate may be prepared by enzymatically hydrolysing the whey fraction in one or more steps. If the whey fraction used as the starting material is substantially lactose free, it is found that the protein suffers much less lysine blockage during the hydrolysis process. This enables the extent of lysine blockage to be reduced from about 15% by weight of total lysine to less than about 10% by weight of lysine; for example about 7% by weight of lysine which greatly improves the nutritional quality of the protein source.

The infant formula may contain a carbohydrate source. Any carbohydrate source conventionally found in infant formulae such as lactose, saccharose, maltodextrin, starch and mixtures thereof may be used although the preferred source of carbohydrates is lactose. Preferably the carbohydrate sources contribute between 35 and 65% of the total energy of the formula.

The infant formula may contain a source of lipids. The lipid source may be any lipid or fat which is suitable for use in infant formulas. Preferred fat sources include palm olein, high oleic sunflower oil and high oleic safflower oil. The essential fatty acids linoleic and α-linolenic acid may also be added as may small amounts of oils containing high quantities of preformed arachidonic acid and docosahexaenoic acid such as fish oils or microbial oils. In total, the fat content is preferably such as to contribute between 30 to 55% of the total energy of the formula. The fat source preferably has a ratio of n-6 to n-3 fatty acids of about 5:1 to about 15:1; for example about 8:1 to about 10:1.

The infant formula may also contain all vitamins and minerals understood to be essential in the daily diet and in nutritionally significant amounts. Minimum requirements have been established for certain vitamins and minerals. Examples of minerals, vitamins and other nutrients optionally present in the infant formula include vitamin A, vitamin B1, vitamin B2, vitamin B6, vitamin B12, vitamin E, vitamin K, vitamin C, vitamin D, folic acid, inositol, niacin, biotin, pantothenic acid, choline, calcium, phosphorous, iodine, iron, magnesium, copper, zinc, manganese, chloride, potassium, sodium, selenium, chromium, molybdenum, taurine, and L-carnitine. Minerals are usually added in salt form. The presence and amounts of specific minerals and other vitamins will vary depending on the intended infant population.

If necessary, the infant formula may contain emulsifiers and stabilisers such as soy lecithin, citric acid esters of mono- and di-glycerides.

The infant formula preferably further contains at least one prebiotic in an amount of 0.3 to 10%. A prebiotic is a non-digestible food ingredient that beneficially affects the host by selectively stimulating the growth and/or activity of one or a limited number of bacteria in the colon, and thus improves host health. Such ingredients are non-digestible in the sense that they are not broken down and absorbed in the stomach or small intestine and thus pass intact to the colon where they are selectively fermented by the beneficial bacteria. Examples of prebiotics include certain oligosaccharides, such as fructooligosaccharides (FOS) and galactooligosaccharides (GOS). A combination of prebiotics may be used such as 90% GOS with 10% short chain fructo-oligosaccharides such as the product sold under the trade mark Raftilose® or 10% inulin such as the product sold under the trade mark Raftiline®. A particularly preferred combination of prebiotics is 70% short chain fructo-oligosaccharides and 30% inulin.

The infant formula may also comprise at least one probiotic bacterial strain. A probiotic is a microbial cell preparation or components of microbial cells with a beneficial effect on the health or well-being of the host. Suitable probiotic bacterial strains include *Lactobacillus rhamnosus* ATCC 53103 obtainable from Valio Oy of Finland under the trade mark LGG, *Lactobacillus rhamnosus* CGMCC 1.3724, *Lactobacillus paracasei* CNCM I-2116, the strain of *Lactobacillus reuteri* sold by BioGaia A.B under the trade mark Reuteri, *Streptococcus salivarius* DSM 13084 sold by BLIS Technologies Limited of New Zealand under the designation K12, *Bifidobacterium lactis* CNCM I-3446 sold *inter alia* by the Christian Hansen company of Denmark under the trade mark Bb12, *Bifidobacterium longum* ATCC BAA-999 sold by Morinaga Milk Industry Co. Ltd. of Japan under the trade mark BB536, the strain of *Bifidobacterium breve* sold by Danisco under the trade mark Bb-03, the strain of *Bifidobacterium breve* sold by Morinaga under the trade mark M-16V, the strain of Bifidobacterium infantis sold by Procter & Gamble Co. under the trade mark Bifantis and the strain of *Bifidobacterium breve* sold by Institut Rosell (Lallemand) under the trade mark R0070 in an amount between 10e3 and 10e12 cfu/g powder, more preferably between 10e7 and 10e12 cfu/g powder.

The infant formula may optionally contain other substances which may have a beneficial effect such as lactoferrin, nucleotides or nucleosides.

The infant formula may be prepared in any suitable manner. For example, they may be prepared by blending together the protein, the carbohydrate source, and the fat source in appropriate proportions. If used, the emulsifiers may be included at this point. The vitamins and minerals may be added at this point but are usually added later to avoid thermal degradation. Any lipophilic vitamins, emulsifiers and the like may be dissolved into the fat source prior to blending. Water, preferably water which has been subjected to reverse osmosis, may then be mixed in to form a liquid mixture. The temperature of the water is conveniently about 50°C to about 80°C to aid dispersal of the ingredients. Commercially available liquefiers may be used to form the liquid mixture. The N-acetyl-lactosamine and/or an oligosaccharide containing N-acetyl-lactosamine will be added at this stage if the final product will be in liquid form. If the final product is to be a powder, the oligosaccharides may likewise be added at this stage if desired. The liquid mixture is then homogenised; for example in two stages.

The liquid mixture may then be thermally treated to reduce bacterial loads, by rapidly heating the liquid mixture to a temperature in the range of about 80°C to about 150°C for about 5 seconds to about 5 minutes, for example. This may be carried out by steam injection, autoclave or by heat exchanger; for example a plate heat exchanger.

Then, the liquid mixture may be cooled to about 60°C to about 85°C; for example by flash cooling. The liquid mixture may then be again homogenised; for example in two stages at about 10 MPa to about 30 MPa in the first stage and about 2 MPa to about 10 MPa in the second stage. The homogenised mixture may then be further cooled to add any heat sensitive components; such as vitamins and minerals. The pH and solids content of the homogenised mixture are conveniently adjusted at this point.

The homogenised mixture is transferred to a suitable drying apparatus such as a spray drier or freeze drier and converted to powder. The powder should have a moisture content of less than about 5% by weight. The oliogsaccharide may be added at this stage by dry-mixing along with the probiotic bacterial strain(s) if used

If a liquid product is preferred, the homogenised mixture may be sterilised then aseptically filled into suitable containers or may be first filled into the containers and then retorted.

In another embodiment, the composition may be a supplement including an oligosaccharide selected from the group consisting of lacto-N-tetraose, lacto-N-neotetraose, lacto-N-hexaose, lacto-N-neohexaose, para-lacto-N-hexaose, para-lacto-N-neohexaose, lacto-N-octaose, lacto-N-neooctaose, iso- lacto-N-octaose, para- lacto-N-octaose and lacto-N-decaose in an amount sufficient to achieve the desired effect. Preferably the daily dose of the oligosaccharide is between 0.1 and 3g. The amount of oligosaccharide to be included in the supplement will be selected accordingly depending upon how the supplement is to be administered. For example, if the supplement is to be administered twice a day, each supplement may contain between 0.05 and 1.5g. The supplement should be in a form suitable for administration to neonatal infants and will contain only those adjuvants and excipients suitable for this age group.

The invention will now be further illustrated by reference to the following example:-

### Example 1

An example of the composition of a suitable infant formula to be used in the present invention is given below

| Nutrient | Per 100kcal | per litre |
|---|---|---|
| Energy (kcal) | 100 | 670 |
| Protein (g) | 1.83 | 12.3 |
| Fat (g) | 5.3 | 35.7 |
| Linoleic acid (g) | 0.79 | 5.3 |
| α-Linolenic acid (mg) | 101 | 675 |
| Lactose (g) | 11.2 | 74.7 |
| Minerals (g) | 0.37 | 2.5 |
| Na (mg) | 23 | 150 |
| K (mg) | 89 | 590 |
| Cl (mg) | 64 | 430 |
| Ca (mg) | 62 | 410 |
| P (mg) | 31 | 210 |
| Mg (mg) | 7 | 50 |
| Mn (µg) | 8 | 50 |
| Se (µg) | 2 | 13 |
| Vitamin A (µg RE) | 105 | 700 |
| Vitamin D (µg) | 1.5 | 10 |
| Vitamin E (mg TE) | 0.8 | 5.4 |
| Vitamin K1 (µg) | 8 | 54 |
| Vitamin C (mg) | 10 | 67 |
| Vitamin B1 (mg) | 0.07 | 0.47 |
| Vitamin B2 (mg) | 0.15 | 1.0 |
| Niacin (mg) | 1 | 6.7 |
| Vitamin B6 mg) | 0.075 | 0.50 |
| Folic acid (µg) | 9 | 60 |
| Pantothenic acid (mg) | 0.45 | 3 |
| Vitamin B12 | 0.3 | 2 |
| Biotin (µg) | 2.2 | 15 |
| Choline (mg) | 10 | 67 |
| Fe (mg) | 1.2 | 8 |
| I (µg) | 15 | 100 |
| Cu (mg) | 0.06 | 0.4 |
| Zn (mg) | 0.75 | 5 |
| LNnT (mg) | 37 | 250 |

## Claims

1. The use of an oligosaccharide selected from the group consisting of lacto-N-tetraose, lacto-N-neotetraose, lacto-N-hexaose, lacto-N-neohexaose, para-lacto-N-hexaose, para-lacto-N-neohexaose, lacto-N-octaose, lacto-N-neooctaose, iso-lacto-N-octaose, para-lacto-N-octaose and lacto-N-decaose in the manufacture of a medicament for infants or a therapeutic nutritional composition for infants for modulating the immune system of a neonatal infant to promote the development in the first few weeks of the life of the infant of a beneficial intestinal microbiota comparable with that found in breast fed infants, wherein the medicament or therapeutic nutritional composition is administered to the infant immediately after delivery and thereafter for at least 2 months.

2. The use of an oligosaccharide selected from the group consisting of lacto-N-tetraose, lacto-N-neotetraose, lacto-N-hexaose, lacto-N-neohexaose, para-lacto-N-hexaose, para-lacto-N-neohexaose, lacto-N-octaose, lacto-N-neooctaose, iso-lacto-N-octaose, para-lacto-N-octaose and lacto-N-decaose in the manufacture of a medicament or therapeutic nutritional composition for administration to a neonatal infant for reducing the risk of subsequent development of allergy in the infant, wherein the medicament or therapeutic nutritional composition is administered to the infant immediately after delivery and thereafter for at least 2 months.

3. The use of Claim 1 or 2, wherein the oligosaccharide is lacto-N-tetraose or lacto-N-neotetraose.

4. The use of any preceding claim, wherein the therapeutic nutritional composition is an infant formula.

5. The use of Claim 4 wherein the infant formula contains from 0.1 to 3g of an oligosaccharide selected from the group consisting of lacto-N-tetraose, lacto-N-neotetraose, lacto-N-hexaose, lacto-N-neohexaose, para-lacto-N-hexaose, para-lacto-N-neohexaose, lacto-N-octaose, lacto-N-neooctaose, iso-lacto-N-octaose, para-lacto-N-octaose and lacto-N-decaose per 100g formula.

6. The use of Claim 4 or 5, where the infant formula further comprises a probiotic bacterial strain in an amount of from 10e3 and 10e12 cfu/g formula on a dry weight basis.

7. The use of any of Claims 4 to 6, wherein the infant formula further comprises at least one prebiotic in an amount of from 0.3 to 10% by weight of the formula.

8. The use of any of Claims 1 to 3 wherein the medicament is a supplement which comprises from 0.1 to 3g of an oligosaccharide selected from the group consisting of lacto-N-tetraose, lacto-N-neotetraose, lacto-N-hexaose, lacto-N-neohexaose, para-lacto-N-hexaose, para-lacto-N-neohexaose, lacto-N-octaose, lacto-N-neooctaose, iso-lacto-N-octaose, para-lacto-N-octaose and lacto-N-decaose per daily dose.

## Patentansprüche

1. Verwendung eines Oligosaccharids, ausgewählt aus der Gruppe aufweisend Lacto-N-tetraose, Lacto-N-neotetraose, Lacto-N-hexaose, Lacto-N-neohexaose, para-Lacto-N-hexaose, para-Lacto-N-neohexaose, Lacto-N-octaose, Lacto-N-neooctaose, iso-Lacto-N-octaose, para-Lacto-N-octaose und Lacto-N-decaose, bei der Herstellung eines Medikamentes für Säuglinge oder einer therapeutischen Nahrungszusammensetzung für Säuglinge zur Modulation des Immunsystems eines Neugeborenen, um die Entwicklung einer günstigen Darmmikrobiota, vergleichbar mit der von gestillten Säuglingen, in den ersten Lebenswochen des Säuglings zu begünstigen, wobei das Medikament oder die therapeutische Nahrungszusammensetzung dem Säugling unmittelbar nach der Geburt und danach für mindestens 2 Monate verabreicht wird.

2. Verwendung eines Oligosaccharids, ausgewählt aus der Gruppe aufweisend Lacto-N-tetraose, Lacto-N-neotetraose, Lacto-N-hexaose, Lacto-N-neohexaose, para-Lacto-N-hexaose, para-Lacto-N-neohexaose, Lacto-N-octaose, Lacto-N-neooctaose, iso-Lacto-N-octaose, para-Lacto-N-octaose und Lacto-N-decaose, bei der Herstellung eines Medikamentes oder einer therapeutischen Nahrungszusammensetzung zur Verabreichung an ein Neugeborenes, um das Risiko der anschließenden Entwicklung einer Allergie bei dem Säugling zu reduzieren, wobei das Medikament oder die therapeutische Nahrungszusammensetzung dem Säugling unmittelbar nach der Geburt und danach für mindestens 2 Monate verabreicht wird.

3. Verwendung nach Anspruch 1 oder 2, wobei das Oligosaccharid Lacto-N-tetraose oder Lacto-N-neotetraose ist.

4. Verwendung nach einem der vorhergehenden Ansprüche, wobei die therapeutische Nahrungszusammensetzung eine Säuglingsnahrung ist.

5. Verwendung nach Anspruch 4, wobei die Säuglingsnahrung von 0,1 bis 3 g eines Oligosaccharids aufweist, ausgewählt aus der Gruppe aufweisend Lacto-N-tetraose, Lacto-N-neotetraose, Lacto-N-hexaose, Lacto-N-neohexaose, para-Lacto-N-hexaose, para-Lacto-N-neohexaose, Lacto-N-octaose, Lacto-N-neooctaose, iso-Lacto-N-octaose, para-Lacto-N-octaose und Lacto-N-decaose je 100 g der Säuglingsnahrung.

6. Verwendung nach Anspruch 4 oder 5, wobei die Säuglingsnahrung weiterhin einen probiotischen Bakterienstamm in einer Mengen von 10e3 und 10e12 KbE/g der Säuglingsnahrung, bezogen auf die Trockenmasse, aufweist.

7. Verwendung nach Anspruch 4 bis 6, wobei die Säuglingsnahrung weiterhin mindestens ein Präbiotikum in einer Menge von 0,3 bis 10 Gew.-% der Säuglingsnahrung aufweist.

8. Verwendung nach einem der Ansprüche 1 bis 3, wobei das Medikament ein Nahrungsergänzungsmittel ist, das von 0,1 bis 3 g eines Oligosaccharids aufweist, das aus der Gruppe ausgewählt ist, aufweisend Lacto-N-tetraose, Lacto-N-neotetraose, Lacto-N-hexaose, Lacto-N-neohexaose, para-Lacto-N-hexaose, para-Lacto-N-neohexaose, Lacto-N-octaose, Lacto-N-neooctaose, iso-Lacto-N-octaose, para-Lacto-N-octaose und Lacto-N-decaose pro Tagesdosis.

## Revendications

1. Utilisation d'un oligosaccharide choisi parmi le groupe constitué de lacto-N-tétraose, lacto-N-néotétraose, lacto-N-hexaose, lacto-N-néohexaose, para-lacto-N-hexaose, para-lacto-N-néohexaose, lacto-N-octaose, lacto-N-néooctaose, iso-lacto-N-octaose, para-lacto-N-octaose et lacto-N-décaose dans la fabrication d'un médicament pour nourrissons ou d'une composition nutritionnelle thérapeutique pour nourrissons pour avoir un effet sur le système immunitaire d'un nourrisson nouveau-né afin de promouvoir le développement, au cours des quelques premières semaines de vie du nourrisson, d'un microbiote intestinal bénéfique comparable à celui constaté chez des nourrissons allaités au sein, dans laquelle le médicament ou la composition nutritionnelle thérapeutique est administré au nouveau-né immédiatement après l'accouchement et par la suite pendant au moins 2 mois.

2. Utilisation d'un oligosaccharide choisi parmi le groupe constitué de lacto-N-tétraose, lacto-N-néotétraose, lacto-N-hexaose, lacto-N-néohexaose, para-lacto-N-hexaose, para-lacto-N-néohexaose, lacto-N-octaose, lacto-N-néooctaose, iso-lacto-N-octaose, para-lacto-N-octaose et lacto-N-décaose dans la fabrication d'un médicament ou d'une composition nutritionnelle thérapeutique pour une administration à un nourrisson nouveau-né pour réduire le risque de développement ultérieur d'allergie chez le nourrisson, dans laquelle le médicament ou la composition nutritionnelle thérapeutique est administré au nouveau-né immédiatement après l'accouchement et par la suite pendant au moins 2 mois.

3. Utilisation selon la revendication 1 ou 2, dans laquelle l'oligosaccharide est du lacto-N-tétraose ou du lacto-N-néotétraose.

4. Utilisation selon l'une des revendications précédentes, dans laquelle la composition nutritionnelle thérapeutique est une préparation pour nourrissons.

5. Utilisation selon la revendication 4, dans laquelle la préparation pour nourrissons contient de 0,1 à 3 g d'un oligosaccharide choisi parmi le groupe constitué de lacto-N-tétraose, lacto-N-néotétraose, lacto-N-hexaose, lacto-N-néohexaose, para-lacto-N-hexaose, para-lacto-N-néohexaose, lacto-N-octaose, lacto-N-néooctaose, iso-lacto-N-octaose, para-lacto-N-octaose et lacto-N-décaose pour 100g de la préparation.

6. Utilisation selon la revendication 4 ou 5, dans laquelle la préparation pour nourrissons comprend en outre une souche bactérienne probiotique dans une quantité comprise entre 10e3 et 10e12 3 ufc/g de préparation sur la base du poids sec.

7. Utilisation selon l'une des revendications 4 à 6, dans laquelle la préparation pour nourrissons comprend en outre au moins un prébiotique dans une quantité comprise entre 0,3 à 10% en poids de la préparation.

8. Utilisation selon l'une quelconque des revendications 1 à 3, dans laquelle le médicament est un complément alimentaire qui comprend entre 0,1 et 3g d'un oligosaccharide choisi parmi le groupe constitué de lacto-N-tétraose, lacto-N-néotétraose, lacto-N-hexaose, lacto-N-néohexaose, para-lacto-N-hexaose, para-lacto-N-néohexaose, lacto-N-octaose, lacto-N-néooctaose, iso-lacto-N-octaose, para-lacto-N-octaose et lacto-N-décaose par dose quotidienne.
